(19) **Europäisches Patentamt**
**European Patent Office**
**Office européen des brevets**

(11) **EP 1 802 796 B1**

(12) **EUROPEAN PATENT SPECIFICATION**

(45) Date of publication and mention
of the grant of the patent:
**10.03.2010 Bulletin 2010/10**

(51) Int Cl.:
***D02G 3/44*** (2006.01)

(21) Application number: **05809938.3**

(22) Date of filing: **13.10.2005**

(86) International application number:
**PCT/US2005/037260**

(87) International publication number:
**WO 2006/044806 (27.04.2006 Gazette 2006/17)**

(54) **IMPROVED STRINGS FOR RACQUETS**

VERBESSERTE SAITEN FÜR TENNISSCHLÄGER

CORDES AMELIOREES POUR RAQUETTES

(84) Designated Contracting States:
**AT BE BG CH CY CZ DE DK EE ES FI FR GB GR HU IE IS IT LI LT LU LV MC NL PL PT RO SE SI SK TR**

(30) Priority: **20.10.2004 US 969809**

(43) Date of publication of application:
**04.07.2007 Bulletin 2007/27**

(73) Proprietor: **Gore Enterprise Holdings, Inc. Newark, DE 19714-9206 (US)**

(72) Inventors:
• **CHU, Chaokang**
**Hockessin, DE 19707 (US)**

• **GAMBALE, Dean, J**
**Wilmington, DE 19808 (US)**

(74) Representative: **Naismith, Robert Stewart et al**
**Marks & Clerk LLP**
**Aurora**
**120 Bothwell Street**
**Glasgow**
**G2 7JS (GB)**

(56) References cited:
**EP-A- 1 531 454        US-A- 4 187 390**
**US-A1- 2001 035 002    US-B2- 6 528 709**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

## BACKGROUND

The present invention relates to strings for sporting applications, and particularly to strings for racquets such as tennis racquets, badminton racquets, squash racquets, racquetball racquets and the like.

Racquet strings must satisfy competing requirements. In a tennis racquet, for example, the principal requirements are playability and durability and it is difficult to satisfy both requirements in a single racquet string type. String construction and material selection has heretofore required a compromise between acceptable playability and durability.

During play, particularly in tennis, the ball is usually hit with some degree of spin. To generate spin, the strings are brushed against the ball to impart a tangential force to it. This brushing action causes the individual strings to slide over one another and wear against each other. The rubbing action of one string against another as well as the impact of the ball creates notches in the strings at the inter-string contact point. These notches are the primary reason for string breakage; as the notching becomes more severe, the tensile strength of the string weakens and eventually it breaks. The friction between the ball and the string during contact with the string surface also causes some string wear.

Several materials have been used in racquet strings in order to achieve a balance of durability and playability. One material, natural gut, enjoys a reputation for unmatched playability. Unfortunately, gut strings have a short life due to notching and wear. Few recreational players use gut strings because they are expensive and wear quickly. Many synthetic string materials, sizes and constructions have been proposed as alternatives to gut. Such synthetics generally are more durable than gut, but are not as playable.

The most common synthetic material is nylon. Although more modem fibers, such as PEEK and aramid fibers, such as Kevlar® are used in racquet strings, nylon multi-filament strings are generally accepted as among the most playable synthetic materials. Nylon strings demonstrate improved durability over gut, but even nylon strings are subject to frequent breakage by certain players, particularly power hitters and those who hit the ball with a lot of spin.

Nylon strings have been proposed in many mono-filament and multi-filament constructions, the more durable strings being the mono-filaments and the more playable strings being the multi-filaments. Within the range of mono-filaments and multi-filaments there are a variety of constructions that have been used to either tailor the durability or the playability of the string.

Coatings have been proposed to improve the abrasion resistance of strings. For example, strings have been dipped or coated with polytetrafluoroethylene in an attempt to reduce the friction between strings, which causes notching. Other attempts have included adding hard, abrasion resistant coatings to the exterior of a string. Such coatings have generally failed because they are inelastic and do not adhere well to the nylon surface as the string stretches in use.

To improve the durability of nylon strings, the addition of high strength fibers such as aramids to multi-filament constructions has been proposed. However, the addition of stiff aramid fibers to a string matrix dramatically reduces the playability of the strings. Nomex, which has better elastic properties than other aramids, has been added to the core of nylon strings with some success with regards to durability, but with a significant tradeoff with regard to playability.

An accepted measure of playabiltiy is dynamic modulus, which is the ratio between the increase of tension and the elongation of a string caused by dynamic impact. This is a measure of how stiff a string is under dynamic conditions similar to that of being struck with a tennis ball during play. To be playable, a racquet string must show elastic properties under dynamic conditions and deform under a given impact. Strings with low dynamic modulus are less stiff and therefore have better playability than strings with a high dynamic modulus, which do not stretch as much and therefore feel stiff. Gut strings may have a dynamic modulus of as low as 17-26 kN/m. In contrast, high strength fibers such as Kevlar may have a dynamic modulus of 88 kN/m to 140 kN/m or more. Nylon strings have a dynamic modulus in a range of about 25 kN/m to about 45 kN/m.

Another factor affecting both durability and playabiltiy is string size, or gauge. For example, a 16 gauge string generally has a larger diameter than a 17 gauge string. Accordingly, the 16 gauge string may last longer. But string size is critical to playability, and thinner strings play better.

Higher gauge strings or thinner diameter strings play better in part because they are more effective at imparting spin to a ball, such as a tennis ball, because thin strings cut deeply into the felt cover of a tennis ball, gripping it to impart the spin necessary for player control. Thicker strings do not penetrate the ball cover as deeply. Thinner strings also deflect more for a given impact. This increase in deflection reduces the shock that the player feels and returns more energy to the ball giving the player more power. Furthermore, thick strings increase wind resistance to racquet swing to a surprising degree.

While the dynamic modulus of an individual string is indicative of its playability, racquets are actually strung with a crossed pattern of strings called a string bed. The strings extending from top to bottom of the racquet head are called the main strings, while those crossing the racquet head are called the cross strings. When the strings move within the string bed, the main strings slide and rub against the cross strings. The resultant friction between strings causes energy loss. This energy loss may also affect playability.

US 2001 035 002 (Carr) teaches an abrasion-resistant composite-coated string for sports racquets and fishing equipment, which includes a single filament or bundle of multifilament and a composite coating. The composite coating is a matrix of material and particles comprising PTFE. The PFTE particles are interspersed within the matrix and within a range of about 11% to 20%.

## SUMMARY

The present invention includes a string for racquets according to claim 1 and a method for making the same according to claim 37.

The string of the present invention may employ a conventional string, such as a string having a center core comprising gut or synthetic material such as nylon, and a polymer cover impregnated with adhesive. The adhesive may be low temperature adhesive. The polymer cover covers the string along at least a portion of the length of the string. As the term "adhesive" is used herein it is intended to mean a material that will form a bond between the polymer cover and the base string. As the term "low temperature adhesive" is used herein it is intended to designate any adhesive that will either form a bond when processed at a temperature less than about 300°C. More preferably, the low temperature adhesive comprises any adhesive that will either cure or form a durable bond at less than about 275, 250, 225, 200, 175, 150, 125,100, 75, 50, or 25°C.

In another embodiment of the invention, at least some of the porosity is filled with an adhesive by applying the adhesive to one or more surfaces of the polymer cover. In an alternative embodiment of the invention, at least some of the porosity is filled, for example, by imbibing or impregnating the porous polymer cover, with adhesive.

In a still further embodiment, the adhesive is applied to at least one surface of the polymer cover and at least some of the porosity is filled with an adhesive.

In an alternative embodiment of the invention, the adhesive is a low temperature adhesive.

In yet another embodiment of the invention, a suitable low temperature adhesive can be applied to at least one surface of the polymer cover and the low temperature adhesive may form a durable bond between the string and cover material. In this embodiment, the adhesive may be continuous or discontinuous.

In order to provide the highest compatibility with a wide variety of underlying string materials, it may be desirable to provide an adhesive material that can be applied, and if necessary cured, at or near room temperature, such as through use of pressure sensitive adhesives, radiation curable adhesives, or the like. Thus, in another embodiment of the invention an adhesive is provided that is cured through exposure to ultraviolet light (hereinafter "UV" light) or an electron beam (hereinafter "EB").

In yet another embodiment of the invention, the polymer cover comprises ePTFE.

In another embodiment, the composite has a thickness of less than about 5% of the racquet string diameter. Preferably, the composite has a thickness of less than about 3% of the racquet string diameter. Most preferably, the composite has a thickness of less than about 1% of the racquet string diameter.

In still another embodiment, the string is of monofiliment construction. Preferably, the string is of multifilament construction. In this embodiment, the filaments of substantially the same diameter are preferred.

In another embodiment, the racquet string of the present invention includes a base string constructed of Nylon, PEEK or gut.

In a still further embodiment, the invention provides for an adhesive comprising at least one filler material. In this embodiment, the filler material may be selected from the group consisting of ceramics, metals, metal coated fillers, metallized fillers, inorganic oxides, carbon, pigments, lubricants and polymers.

In another embodiment, the adhesive comprises a urethane acrylate or a cationic epoxy.

In yet another embodiment of the invention, the cover is helically wrapped around the base string.

In another embodiment the invention is a racquet string having a diameter of less than about 1.34 mm and having dynamic modulus of less than about 30kN/m and a durability of at least about 2200.

In a still further embodiment, the invention is a racquet string having a diameter of less than about 1.25 mm and having dynamic modulus of less than about 30kN/m and a durability of at least about 500 and at least about 1000.

In a yet another embodiment, the invention is a racquet string having a diameter of less than about 1.20 mm and having dynamic modulus of less than about 30 kN/m and a durability of at least about 500 and at least about 800.

## DESCRIPTION OF THE DRAWINGS

The operation of the present invention should become apparent from the following description when considered in conjunction with the accompanying drawings, in which:

Figure 1 is a three-quarter perspective view of a racquet;
Figure 2 is a schematic drawing of a porous film of the invention wherein at least some of the porosity of the film is filled with adhesive;

Figure 3 is a schematic drawing of a porous film of the invention wherein substantially all of the porosity of the film is filled with adhesive;

Figure 4 is a schematic drawing of a porous film of the invention wherein at least some of the porosity of the film is filled with adhesive and one surface of the film is provided with a relatively thin layer of adhesive;

Figure 5 is a schematic drawing of a porous film of the invention wherein substantially all of the porosity of the film is filled with adhesive and one surface of the film is provided with a relatively thin layer of adhesive;

Figure 6 is a schematic drawing of a porous film of the invention wherein substantially all of the porosity of the film is filled with adhesive and both surfaces of the film are provided with a relatively thin layer of adhesive;

Figure 7 is a schematic drawing of a porous film of the invention wherein at least some of the porosity of the film is filled with adhesive, but the adhesive is not coincident with the surfaces of the film;

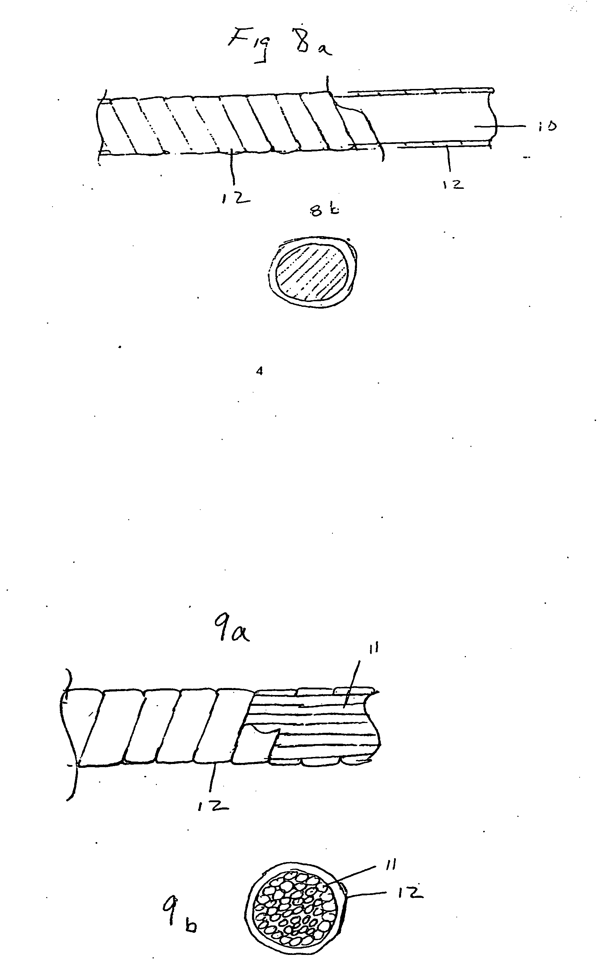

Figures 8a through 9b show string constructions according to the invention.

Figure 10 is a perspective view of the apparatus used to determine string durability.

Figure 11 is a schematic diagram of the apparatus used to determine elastic modulus of strings.

Figure 12 is a perspective view of the apparatus used to determine the elastic modulus of strings.

## DETAILED DESCRIPTION OF THE INVENTION

The present invention relates generally to improved racquet strings.

The invention solves the problem of string durability without diminishing the playability of the string. This is accomplished by wrapping (or otherwise covering) at least a portion of the string with a polymer cover The cover should be sufficiently durable to withstand abrasion occasioned by ball impact and string movement.

The polymer cover has at least some porosity. As used herein, "porosity" refers to the property or state of a material having voids or interstices. The cover may be impregnated with an adhesive by applying adhesive to one or more surfaces of the polymer cover. By utilizing a polymer cover comprising at least some porosity, at least some of the porosity is filled with the adhesive.

This novel construction uniquely combines the hardness of a durable adhesive with the lubricity of a polymer having a low coefficient of friction. A polymer string cover alone may not provide adequate abrasion resistance; however, the inventors have found that by filling at least some porosity of the polymer cover with adhesive, abrasion resistance may be significantly improved, while playability is retained. In this way, the porous polymer provides a lubricious matrix that supports the highly abrasion resistant adhesive. The abrasive resistant adhesive is bound within this matrix and, despite its hardness, does not flake from the flexible string. Moreover, the lubricious polymeric membrane reduces friction between strings. Reducing friction at the intersection of strings may further improve playability by improving energy return, and further reduces string breakage by inhibiting notches.

In an aspect of the invention, a suitable low temperature adhesive can be applied to at least one surface of the polymer cover and the low temperature adhesive may form a durable bond between the string and cover material.

The porous polymer cover of the present invention improves durability by providing a wear-resistant surface, but avoids the problem of restricting elongation or movement of the string. Moreover, by filling at least some, or substantially all, of the porosity of the cover with adhesive, durability of the string is further improved.

It has been discovered that the porous polymer can be altered to withstand substantial wear and abrasion during use. Wear and abrasion resistance can be improved by, for example, careful selection of the adhesive used, the addition of certain filler materials, as well as the amount of porosity filled with the adhesive. Thus, by careful selection of adhesive type, amount of adhesive used, and filler materials (if used), an extremely durable and abrasion resistant cover can be fabricated to inhibit notching.

The present invention also solves the problem of string contamination. In applications such as tennis, grass and clay courts in particular may expose the string to contaminants. The polymer cover protects the core from abrasive contaminants that contribute to premature wear, such as clay and silica which are transferred from the court surface to the string by ball impact.

Materials suitable for use in the porous polymer cover of the present invention include, but are not limited to, the following fluoropolymers: polytetrafluoroethylene (PTFE), particularly porous expanded PTFE (ePTFE); fluorinated ethylene propylene (FEP); polyethylene, including ultrahigh molecular weight polyethylene; perfluoro alkoxy resin (PFA); polyurethane; polypropylene; polyester, polyimide; and polyamide.

Although the invention includes use of any porous polymer cover materials, particularly preferred are porous fluoropolymer films, with PTFE and ePTFE being even more preferred. The porosity of the porous polymer cover can be either partially or substantially filled with adhesive. For example, a relatively small amount of adhesive can be supplied to a select portion of the film porosity, while leaving most of the porosity of the film unfilled. In an aspect of the invention, adhesive can be evenly distributed throughout the porosity of the cover from one side of the cover to the other side, while still leaving at least some porosity unfilled. Moreover, in a further aspect of the invention, substantially all of the porosity of the film can be filled with adhesive to perhaps result in better abrasion resistance and better adhesion.

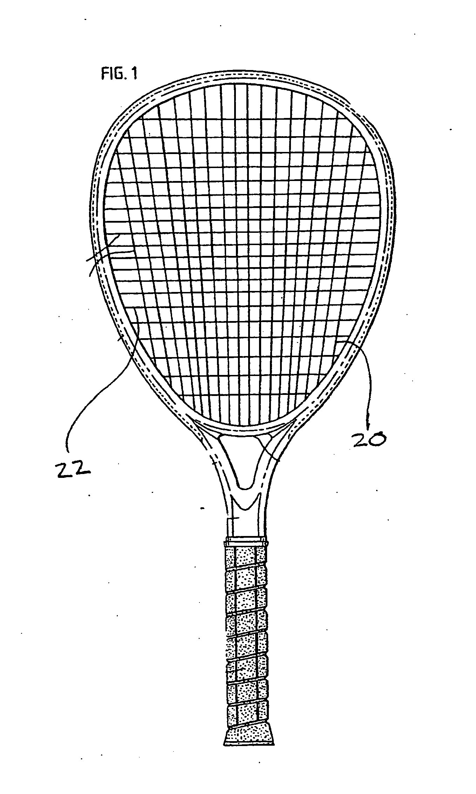

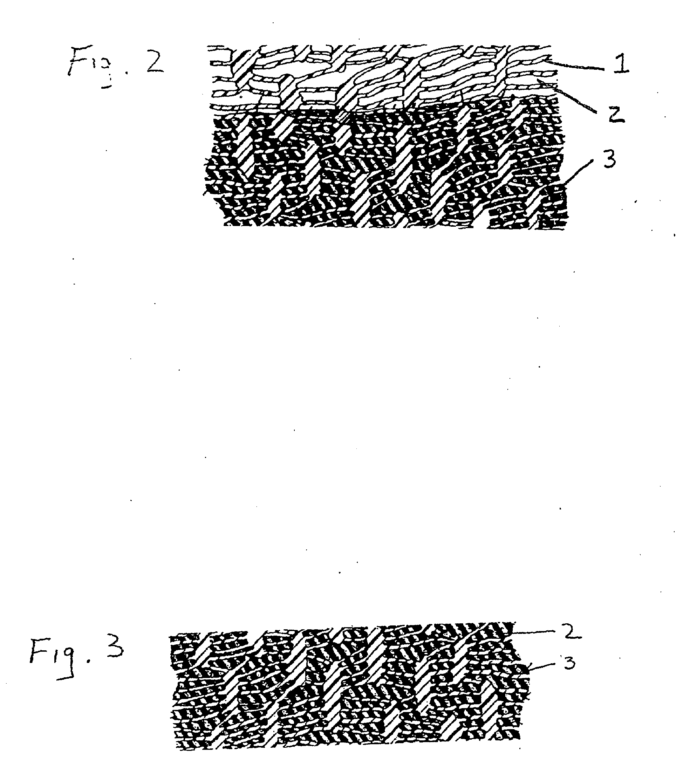

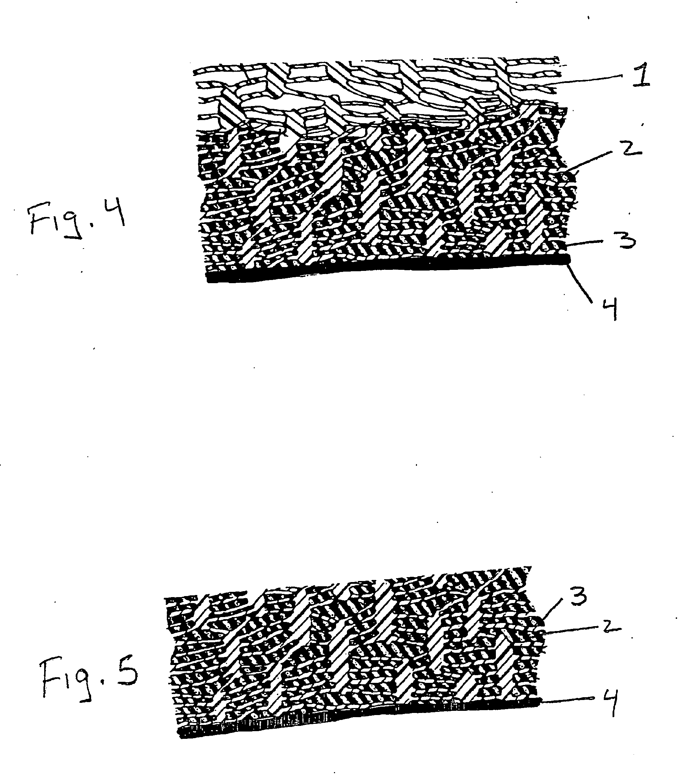

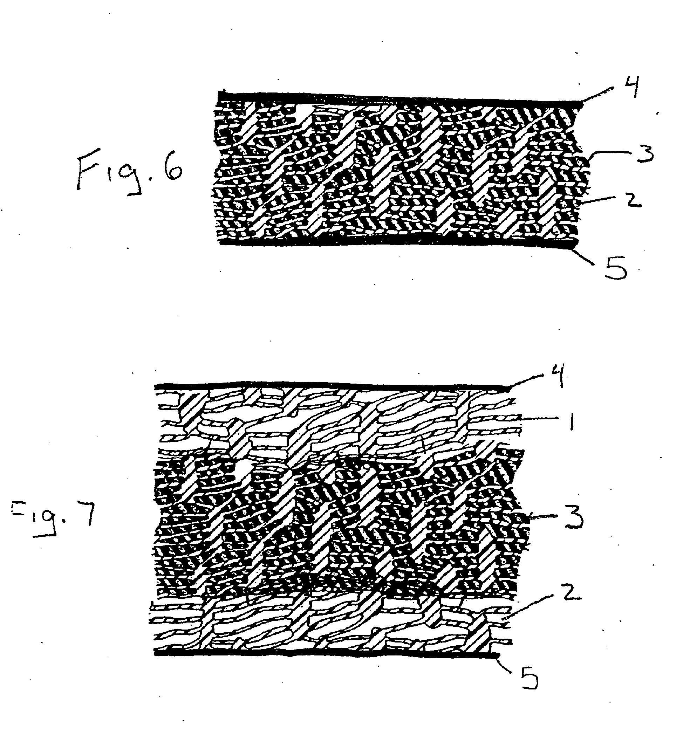

Turning to the figures, Figure 1 shows a tennis racquet with main strings (20) and cross strings (22). Figure 2 illustrates a porous cover material 1, where at least some of the porosity 2 is filled with adhesive 3. Figure 3 illustrates a porous cover where substantially all of the porosity 2 is filled with adhesive 3. Figure 4 illustrates an aspect of the invention wherein at least some of the porosity 2 is filled with adhesive 3 and an additional surface layer of adhesive 4 is supplied to one surface of the film. Figure 5 illustrates an aspect of the invention where substantially all of the porosity 2 has been filled with adhesive 3 and an additional surface layer of adhesive 4 is supplied to one surface of the film. Figure 6 illustrates an aspect of the invention where substantially all of the porosity 2 has been filled with adhesive 3 and both surfaces of the cover are supplied with a surface layer of adhesive 4 and 5. Figure 7 illustrates an embodiment in which some of the porosity 2 is filled with adhesive 2 and both sides of the film are supplied with a surface layer of adhesive. Although covers with any amount of porosity may be used, preferably the cover has a bulk density about 0.7 g/cc, before filling with adhesive.

A preferred cover material is a porous fluoropolymer material such as uniaxially expanded PTFE. This material has demonstrated exceptional durability without affecting the playability of the base string. Porous expanded PTFE, such as that made in accordance with United States Patent Nos. 3,953,566; 3,962,153; 4,096,227; and 4,187,390, comprises a porous network of polymeric nodes and interconnecting fibrils. These kinds of material are commercially available in a variety of forms from W. L. Gore & Associates, Inc., Newark, DE.

Expanded PTFE is formed when PTFE is heated and rapidly expanded by stretching in at least one direction in the manner described in the above listed patents. The resulting expanded PTFE material achieves a number of exceptional properties, including exceptional strength in the direction of expansion, and exceptionally high flexibility, and conformability. The strength properties in both the longitudinal and transverse directions of the ePTFE may be altered in the expansion process, or by other means known in the art to achieve the desired effect or property.

As the term "expanded PTFE" is used herein, it is intended to include any PTFE material having a node and fibril structure, including in the range from a slightly expanded structure having fibrils extending from relatively large nodes of polymeric material, to an extremely expanded structure having very long fibrils interconnected by small nodes. The fibrillar character of the structure is identified by microscopy. While the nodes may easily be identified for some structures, many extremely expanded structures consist almost exclusively of fibrils with very small nodes.

When a porous polymer cover material is used, at least some, or substantially all, of the porosity of the porous polymer cover can be filled with adhesive. Additionally, adhesive can also be provided as a continuous or discontinuous coating on one or both sides of the cover. As used herein, "discontinuous" means that the adhesive does not fully cover the surface of the underlying cover. "Continuous" means without holes or gaps extending through the adhesive coating (i.e. fully covering the surface of the underlying cover). The exact amount of adhesive used depends upon a number of variables. For example, adding more adhesive may further improve durability and abrasion resistance, but may also increase string mass, which may affect playability. Providing less adhesive may result in less durability and reduced abrasion resistance. However, less adhesive may tend to preserve playability: Once the cover has been impregnated or otherwise filled with adhesive, the preferred percent mass of adhesive to ePTFE is 45%.

In order to provide the highest compatibility with a wide variety of underlying string materials, it may be desirable to provide a low temperature adhesive that can be applied, and if necessary cured, at or near room temperature, such as through use of pressure sensitive adhesives or radiation curable adhesives, or the like.

Low temperature adhesives include any adhesive that will either cure or form a durable bond when processed at a temperature of less than about 300°C. Suitable low temperature adhesives include any suitable thermoset resin. For example, suitable thermoset resins include epoxies (including acrylated epoxies), polyurethanes, phenolics, and other thermosets. Suitable thermoplastic resins include, for example, polyethylene, polypropylene, polystyrene, polyvinyl chloride, polyurethanes, and fluoropolymers such as THV (tetrafluoroethylene, hexafluoropropylene, and vinylide fluoride), HTE (hexafluoropropylene, tetrafluoroethylene, and ethylene), EFEP (ethylene tetra fluoro ethylene based copolymer), ETFE (ethylene tetrafluoroethylene), and PVDF (polyvinylidine fluoride), and blends thereof. Other thermoplastic resins are also useful, provided that they are processable at temperatures of less than about 300 C.

Thermally activated adhesives which can cure or form a durable bond when the adhesive is heated, such as THV 220 (tetrafluoroethylene, hexafluoropropylene, and vinylide fluoride, available from Dyneon, LLC) and adhesives which can be caused to cure through chemical reaction, such as known moisture cure adhesives (e.g., polyurethane prepolymers, etc.) or other chemically activated adhesives, can also be used.

In a preferred embodiment, the low temperature adhesive comprises UV-curable adhesive. As used herein, UV-curable is defined as a material that will react under UV light to cure or form a durable bond. The UV light is provided by a lamp with suitable spectral intensity, spectral dosage and wavelength. Those of skill in the art will appreciate that curing with UV light may be carried out at various rates, and that the distance between the sample being cured and the UV lamp can be varied, provided the appropriate spectral dosage is applied. In an aspect of the invention, the UV curable material can also be sensitive to visible light. However, preferred conditions are present only under the UV spectrum (100-400nm). In this range, the underlying core material will not be damaged during the processing of the string. Suitable UV-curable adhesives include, but are not limited to, epoxies, acrylated epoxies, acrylated urethanes, acrylated silicones, acrylated polyethers, acrylated polyester, acrylated polybutadiene, and acrylated fluoropolymers. Specific examples of these adhesives include acrylated aliphatic oligomers, acrylated aromatic oligomers, acrylated epoxy monomers, acrylated epoxy oligomers, aliphatic epoxy acrylates, aliphatic urethane acrylates, aliphatic urethane methacrylates, allyl methacrylate, amine-modified oligoether acrylates, amine-modified polyether acrylates, aromatic acid acrylate, aromatic epoxy acrylates, aromatic urethane methacrylates, butylene glycol acrylate, stearyl acrylate, cycloaliphatic epoxides, cylcohexyl methacrylate, ethylene glycol dimethacrylate, epoxy methacrylates, epoxy soy bean acrylates, glycidyl methacrylate, hexanediol dimethacrylate, isodecyl acrylate, isooctyl acrylate, oligoether acrylates, polybutadiene diacrylate, polyester acrylate monomers, polyester acrylate oligomers, polyethylene glycol dimethacrylate, stearyl methacrylate, triethylene glycol diacetate, and vinyl ethers. Preferred UV-curable adhesives include, for example, urethane acrylates and cationic epoxies.

It may be desirable to utilize a solvent to aid in providing adhesive to the porosity of the porous polymer cover. The ratio of solvent material to adhesive can vary and will also be readily determinable by the skilled artisan. Preferable solvent materials will also be apparent to one skilled in the art and include, for example, alcohols, ketones, etc. A preferred solvent is isopropyl alcohol (IPA). When a solvent material is utilized, the solvent material can be easily removed or driven off once the adhesive is provided to at least some of the porosity of the porous polymer cover as desired.

In a further aspect of the invention, the low temperature adhesive can be combined (e.g., mixed, blended, etc.) with a suitable filler material. Suitable filler materials may include, but are not limited to, ceramics, metals, inorganic oxides, metal coated materials, metallized materials, carbon, pigments and polymers, which can be provided in any suitable form (e.g., particulates, fibers, etc.) Preferably, fillers are in nanoparticle size. Filler materials may be desirable to alter certain properties of the covered string (e.g., to improve abrasion resistance, or to provide color, etc.). Use of solvent may be particularly useful when at least partially filling the porosity of a porous cover with an adhesive/filler material combination.

The adhesive may be applied to the cover by a variety of methods known in the art. With regard to porous polymer covers, suitable adhesive application means include, for example, coating techniques (e.g., dip coating or spray coating), solvent imbibing, vacuum assisted coating, pressure assisted coating, nip coating, and other suitable means which would result in the adhesive filling at least some of the porosity of the porous polymer cover.

As stated above, a preferred porous polymer cover is expanded PTFE. At least a portion of the porosity of the expanded PTFE is filled with low temperature adhesive. In an aspect of the invention, substantially all of the porosity of the expanded PTFE film is filled with low temperature adhesive. Furthermore, one or more surfaces of the expanded PTFE may be provided with a relatively thin surface layer of low temperature adhesive for bonding the cover to the base string. Such surface layer(s) of adhesive can be either continuous or discontinuous. In a preferred embodiment the surface layer(s) of adhesive is a continuous layer. Preferably, the film is impregnated with an adhesive/solvent solution, thus allowing good penetration of the adhesive into the porosity of the film. Impregnating is accomplished by first preparing an adhesive/solvent solution, and second, combining this solution with a porous film like expanded PTFE. Solvents such as alcohols and ketones are capable of dissolving adhesives so that the adhesive can penetrate and occupy the porosity of the porous film. There are many suitable adhesives (e.g., urethanes, epoxies, etc.) that can be dissolved in suitable solvents. In an aspect of the invention, the adhesive is UV-curable urethane-acrylate. This adhesive will also cure by other mechanisms such as through heating and chemical reaction.

The mass of adhesive delivered to the expanded PTFE film (or other polymer cover material) can be regulated by the solvent to adhesive ratio in the solvent/adhesive solution and by the rate at which it is applied. A spreading mechanism can be used to distribute the adhesive/solvent solution after it contacts the film surface. Once the film has accepted the adhesive/solvent solution, or becomes impregnated, the mechanical characteristics of the film can change and it may have the tendency to shrink. In order to stabilize the film, a suitable liner can be provided to the film following this step. An example of a suitable liner material is polyester release film. Another suitable liner material may be a silicone-coated paper. In any event, both the liner and the film can be contacted together and placed into a forced air oven. The heated air can be blown across the flat side of the film oriented with the non-liner side toward the air stream. This drives off the solvent and leaves the adhesive within the porosity of the film. The film can be removed from the liner before applying the film to the string.

Once the low temperature adhesive has been provided to at least one surface of the polymer cover, and the low temperature adhesive has at least partially filled, or is otherwise provided to, the porosity of the cover (and the solvent driven off, if a solvent is used), the cover can then be placed in contact with the string and the low temperature adhesive can then be cured.

The cover of the present invention may be applied in a variety of manners while maintaining the benefits of the present invention. The cover may be wrapped longitudinally (in a "cigarette wrap" manner), or as a continuous and seamless tube surrounding the string. Preferably, the string is helically wrapped with a cover material. In this embodiment, the string may be provided with a cover in the form of a wrapped polymer layer having overlapping edges to form a continuous cover or with non-overlapping edges. The polymer layer may optionally be heated to thermally bond the overlapped edges together. The cover may or may not include an adhesive coating on its outwardly facing surface. The adhesive coating serves to adhere the wraps to the base string and may also provide an additional protective layer to shield the cover from wear and contamination.

Although particularly preferred base string materials include gut or nylon materials, cores comprising other synthetic materials or aramid fibers may also benefit from the use of covers made and applied in accordance with the present invention. However, the covers are particularly attractive when used in combination with highly playable strings, such as nylon or gut strings. Although gut and nylon are typical materials for strings, another preferred material for the string of the invention is PEEK. PEEK strings may provide better durability than nylon and demonstrate acceptable playability.

Regardless of the type of base string, once the string is provided with the cover, the adhesive can be cured to result in the covered string of the invention.

The particular curing mechanism used, such as heat, UV/EB radiation, and chemical reaction, will depend on the type of adhesive used. One preferred adhesive is urethane-acrylate, which is capable of curing via heating and/or UV radiation. The preferred mechanism for curing this adhesive on a gut or synthetic string is UV radiation because of its relatively low temperature application.

As discussed above, prolonged high temperature processes can degrade the performance of strings with gut or synthetic components by compromising the properties of the materials therein. Degraded performance may be observed as a reduction of durability or an increase in dynamic modulus. It is therefore desirable to process strings at temperatures that do not change string performance. Thus, in an aspect of the invention preferred low temperature adhesives include adhesives that bond or can be cured at a temperature of about 150° C or less and, in a further aspect of the invention, at a temperature of about 120° C or less. More preferably, the low temperature adhesive comprises any adhesive that will either cure or form a durable bond at less than about 100, 75, 50, or 25° C.

To cure the adhesive by UV/EB radiation, the covered string can be placed in tension to keep the covered string straight. Important parameters for the UV curing process are spectral intensity of UV light, measured by Watts/cm$^2$, and spectral dosage of UV light, measured by Joules/ cm$^2$. The preferred light intensity, wavelength and dosage depend upon the selection of photoinitiators and the formulation of the adhesive blend, and are readily determined by one of skill in the art. Upon exiting the UV oven, the string should have a tack free surface, indicating that the adhesive has cured.

In an aspect of the invention, a single layer of expanded PTFE, having been stretched in the longitudinal and transverse directions and impregnated with adhesive, is provided to the base string. This is accomplished by helically wrapping the string at a pitch angle measured from the end of the string. This construction is believed to provide excellent strength and durability while maintaining the playability of the base string.

Without intending to limit the scope of the present invention, the following examples illustrate how the present invention may be made and used:

EXAMPLES

Example 1

An example of a string according to the present invention was prepared by helically wrapping a 1.19 mm diameter multi-filament nylon string that was obtained from Prince Mfg. Co. with a polymer film impregnated with UV-curable adhesive. The string was made in the following manner:

Expanded PTFE film with a thickness of about 0.015 mm was obtained from WL Gore and Associates, Inc., Newark, DE. The expanded PTFE film had a bulk density of 0.7 g/cc, and was further characterized by a matrix tensile strength of about 41,000 psi in the longitudinal direction and a Bubble Point of 68 psi.

A 30 wt.% adhesive solution was prepared in isopropyl alcohol for impregnating the expanded PTFE film. The adhesive composition is 60 wt.% aliphatic polyester based urethane diacrylate oligomer blended with ethoxylated trimethylol propane triacrylate (available from Sartomer Company, Exton, PA as CN963E75), 32 wt.% triacrylate acid ester (available from Sartomer Company as CD9052), and 8 wt.% Genocure DMHA, available from Rahn USA Corp., Aurora, IN. This solvent-adhesive solution was dispensed and spread evenly across the expanded PTFE film. A polyester release film grade UV5010 was used as a liner and combined with the film as the solvent-adhesive solution penetrated the expanded PTFE film. Both the liner and impregnated film were sent through an oven (set at about 120°C) to drive off the solvent. The film was removed from the oven and a substantial fully impregnated structure with adhesive coincident with both surfaces of the film and a thin surface coat of adhesive present on the liner side was recovered. The thin surface coat substantially covered the expanded PTFE surface.

The 3.56 mm wide impregnated film was wrapped in a non-overlapping helical fashion around the base string at a pitch angle of 32 degrees while leaving little or no gap between the film layers contacted. The resultant construction was a string with a single layer of impregnated film covering the entire length of the string.

The covered string was fed through a 300 Watt F300S Electrode-less UV Lamp System provided by Fusion UV Systems, Inc., Gaithersburg, MD. The UV lamp was equipped with an H-bulb and F6 light shield for wire/cable applications with 360° reflection. UV dosage to cure the adhesive was controlled by the line speed, which was set to 20 ft/min. Prior to inserting the string, the UV oven was purged with nitrogen to remove oxygen from the oven.

Once each string exited the UV lamp system, it was observed to have a tack-free surface, indicating that the impregnated adhesive had cured. It was further noted that the cover conformed to the string. The covered string diameter was 1.24 mm.

The string was installed in a racquet and was found to have excellent playability (that is, the playability was at least equal to that of comparable diameter multifilament nylon strings as measured by the dynamic modulus). During play, the strings felt smoother and did not require repositioning as frequently as an uncovered string.

Moreover, the durability was significantly improved. During play tests, the strings exhibited noticeably less notching at string contact points. The covered string was also tested on a durability tester, and the durability was reported in Table 1 below. The inventive strings show significant durability improvement over a comparable diameter nylon string, without increasing the dynamic modulus.

Example 2

A second, slightly smaller example string was made according to the present invention by helically wrapping a 1.13 mm diameter multi-filament nylon string that was obtained from Prince Mfg. Co. with a polymer film impregnated with UV-curable adhesive. The string was made in the manner described above in Example 1 by wrapping the base string with the same ePTFE film impregnated with a low temperature adhesive used in Example 1.

The final string diameter was 1.18 mm. As reflected in Table 1, the inventive string shows much better durability than a comparable diameter nylon string, and a similar dynamic modulus.

TEST METHODS

Durability

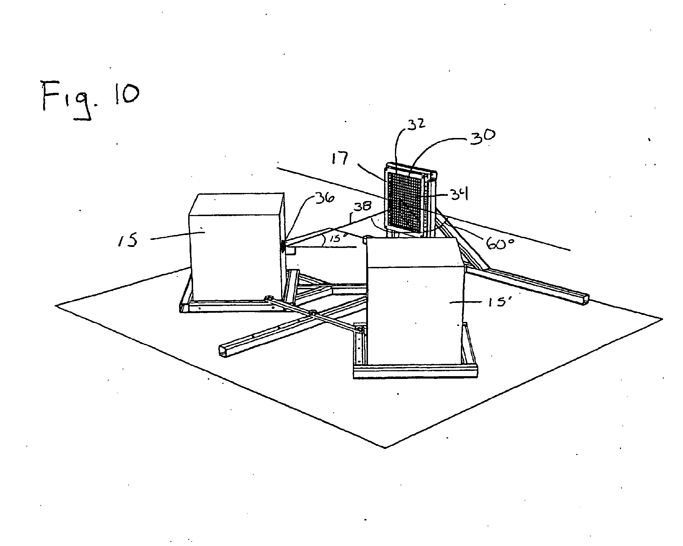

The durability test apparatus is depicted in Figure 10. Tennis balls were alternatively fired at 60 MPH from two ball machines 15, 15' such that the balls contacted a simulated racquet frame 17 at a rate of one every 4 seconds. Ball speed was measured at each ball machine using laser speed recording equipment. The simulated racquet string bed 30 was constructed of an 8-3/4" x 11-1/2" rectangular aluminum frame. The string bed was strung with a pattern of 16 main strings 32 and 18 cross strings 34. Delrin® grommets (not shown) were used to reduce string wear at the frame. The frame was strung at a tension of 58 pounds. The frame was placed perpendicular to the ground such that the discharges 36 of the ball machines were both 25" from the center of the sting bed. The balls traveled along flight path 38 and contacted the string bed at a horizontal angle of 60° and an upward vertical angle of 15°. This was intended to simulate top-spin action.

The test was conducted at an ambient temperature of 20°C. Ten new Tretom Micro X 90 balls were loaded into the ball machines. As each ball machine alternately fired the balls at the string bed, the main strings moved back and forth against the cross strings. After impact, the balls were continuously fed back into the ball machines. The balls were continuously fired from both machines until a string broke. Durability was measured and recorded as the number of impacts at which the string broke.

Dynamic Modulus

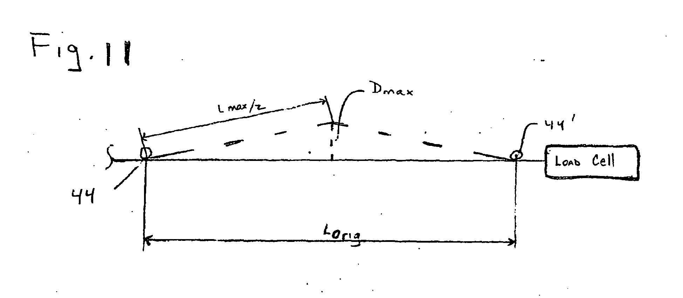

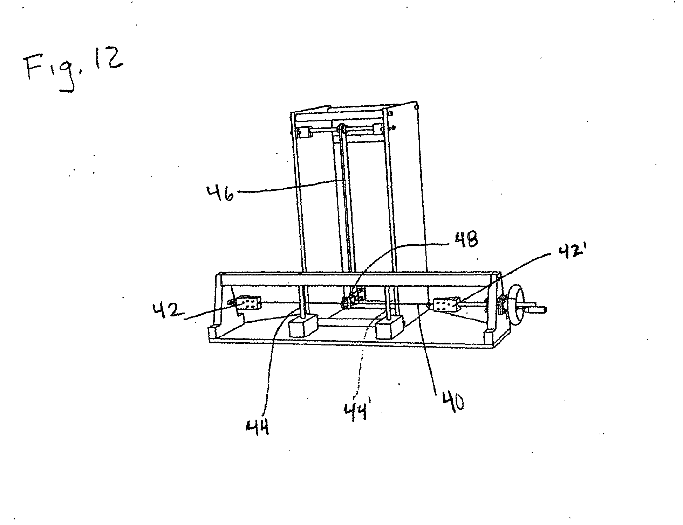

An apparatus for testing the dynamic modulus of a string is depicted in Figure 12 and schematically illustrated in Figure 11. A string sample 40 was held horizontally in metal clamps 42, 42' spaced 340 mm (12.6 in) apart. Two metal bars 44, 44' were positioned between the clamps just contacting the string to support the string. The bar centerline spacing was 300 mm. The string was tensioned at 28 kg. The test was conducted at an ambient temperature of 20°C.

A pendulum 46 was swung into the string to contact the string at the centerpoint of the span between the support bars. The pendulum includes a 0.8" (20.3 mm) flat head hammer face 48, which makes contact with the string. The pendulum weighs 720 g, and its barycenter is 450 mm from the rotation point. At impact, the angular speed is 5.35 rad/s, resulting in a hammer speed of 3.18 m/s.

When the hammer face hits the string, the maximum deflection of the center point of the string was measured using laser measurement equipment. The maximum tension increase in the string was also monitored using a load cell attached to one end of the string. From the maximum deflection ($D_{max}$) and span $L_{Orig}$, the total lengthwise stretch ($\Delta L$) was calculated according to the formula:

$$\Delta L = L_{Max} - L_{Orig}$$

where $L_{orig}$ is the original string length and Lmax is the maximum string length. Maximum string length is determined by the equation:

$$L_{Max} = 2 * \sqrt{\left(\frac{L_{Orig}}{2}\right)^2 + D_{Max}^2}$$

Where $D_{max}$ is the maximum deflection

The dynamic modulus, k, may be calculated by dividing the maximum change in string tension ($\Delta T$) as measured by the load cell by the total lengthwise stretch ($\Delta L$) at impact. Dynamic modulus has units of kN/m.

Bubble Point

The Bubble Point test provides an estimation of maximum pore size. Liquids with surface free energies less than that of stretched porous PTFE can be forced out of the structure with the application of a differential pressure. This clearing will occur from the largest passageways first. A passageway is then created through which bulk air flow can take place. The air flow appears as a steady stream of small bubbles through the liquid layer on top of the sample. The pressure at which the first bulk air flow takes place is called the bubble point and is dependent on the surface tension of the test fluid and the size of the largest opening.

The Bubble Point is measured using the procedures of ASTM F316-86 as guideline. Isopropanol was used as the wetting fluid to fill the pores of the test specimen. The test sample is placed in a filter holder (available from Millipore Corporation, Billerica, Massachusetts), covered with a support screen and the locking ring of the holder attached. The top of the holder is then filled with isopropanol, and the holder is attached to an air supply with a regulated control valve. The holder is placed under a magnifying lens with a light and the air pressure is increased until a continuous stream of bubbles is seen coming through the support screen covered with isopropanol.

The Bubble Point is the pressure of air required to displace the isopropanol from the largest pores of the test specimen and create the first continuous stream of bubbles detectable by their rise through a layer of isopropanol covering the porous media.

Matrix Tensile Strength

Tensile strength of ePTFE materials including ePTFE films is measured using an INSTRON tensile testing machine with pneumatic cord and yam grip jaws. The machine tested 0.25 inch wide samples using a 1 inch jaw separation distance and a crosshead speed of 10 inches/minute. Matrix tensile strength of porous PTFE samples is determined by the formula:

(2.2 g/cc x tensile strength)/density of tested material, where 2.2 g/cc is taken to be the density of non-porous PTFE.

Table 1

|  | String Diameter | Durability (Impacts) | Dynamic Modulus (kN/m) |
|---|---|---|---|
| Comparative Example 1 | 1.24 mm | 398 | 29.66 |
| Inventive Example 1 | 1.24 mm | 1163 | 25.65 |
| Comparative Example 2 | 1.19 mm | 344 | 25.76 |
| Inventive Example 2 | 1.18 mm | 910 | 24.99 |

**Claims**

1.  A string for sporting applications, the string comprising:

    a) a string or core; and
    b) a composite comprising a polymeric membrane (1) having at least some porosity (2) and an adhesive (3) disposed within said at least some porosity, the composite covering at least a portion of said string or core.

2.  The string of claim 1 wherein said at least some porosity (2) is filled with the adhesive (3).

3.  The string of claim 2 further comprising an adhesive layer (4, 5) disposed upon at least one surface of said polymeric membrane (1).

4.  The string of claim 3 wherein the adhesive layer (4, 5) is discontinuous.

5.  The string of claim 3 wherein the adhesive layer (4, 5) is continuous.

6.  The string of claim 1 in which the adhesive (3) comprises a low temperature adhesive.

7.  The string of claim 6 in which the low temperature adhesive is UV cured.

8.  The string of claim 6 wherein the low temperature adhesive further comprises at least one filler material.

9.  The string of claim 8 wherein the at least one filler material comprises at least a material selected from the group consisting of: ceramics, metals, metal coated fillers, metallized fillers, inorganic oxides, carbon, pigments, lubricants and polymers.

10. The string of claim 6 wherein the low temperature adhesive comprises at least a material selected from the group consisting of urethane acrylates and cationic epoxies.

11. The string of claim 1 in which the composite is helically wrapped around at least a portion of the string or core.

12. The string of claim 1 in which the composite is longitudinally wrapped around at least a portion of the string or core.

13. The string of claim 1 wherein the polymeric membrane comprises fluoropolymer.

14. The string of claim 13 wherein the fluoropolymer is expanded polytetrafluoroethylene.

15. The string of claim 1 in which the composite has a thickness of less than about 5 percent of the string or core diameter.

16. The string of claim 1 in which the composite has a thickness of less than about 3 percent of the string or core diameter.

17. The string of claim 1 in which the composite has a thickness of less than about 1 percent of the string or core diameter.

18. The string of claim 1, wherein the string or core comprises natural gut.

19. The string of claim 1, wherein the string or core is a mono-filament.

20. The string of claim 1, wherein the string or core comprises a plurality of filaments.

21. The string of claim 20, wherein said filaments are of substantially the same diameter.

22. The string according to claim 1, the string having a diameter of less than about 1.34 mm and having dynamic modulus of less than about 30kN/m and a durability of at least about 2200.

23. The string according to claim 1, the string having a diameter of less than about 1.25 mm and having dynamic modulus of less than about 30kN/m and a durability of at least about 500.

24. The string according to claim 1, the string having a diameter of less than about 1.25 mm and having dynamic modulus of less than about 30kN/m and a durability of at least about 1000.

25. The string according to claim 1, the string having a diameter of less than about 1.20 mm and having dynamic modulus of less than about 30kN/m and a durability of at least about 500.

26. The string according to claim 1, the string having a diameter of less than about 1.20 mm and having dynamic modulus of less than about 30kN/m and a durability of at least about 800.

27. The string of claim 1, wherein the string or core comprises synthetic fibers.

28. The string of claim 27, wherein the synthetic fibers comprise polyamide.

29. The rstring of claim 28, wherein the synthetic fibers comprise nylon.

30. The string of claim 27, wherein the synthetic fibers comprise polyester.

31. The string of claim 27, wherein the synthetic fibers comprise PEEK.

32. The string according to claim 1, wherein the polymeric membrane (1) is an expanded polytetrafluoroethylene membrane having at least some porosity (2), the adhesive substantially filling said at least some porosity (2), and wherein the racquet string further comprises a continuous adhesive layer (4, 5) disposed upon at least one surface of said expanded polytetrafluoroethylene membrane (1), said composite covering at least a portion of said string or core.

33. The string according to claim 1, wherein the polymeric membrane (1) is an expanded polytetrafluoroethylene membrane having at least some porosity (2), the adhesive substantially filling said at least some porosity (2), and wherein the racquet string further comprises a discontinuous adhesive layer (4,5) disposed upon at least one surface of said expanded polytetrafluoroethylene membrane, said composite covering at least a portion of said string or core.

34. The string according to any of the claims 1 to 33, wherein the string is configured for use with a racquet.

35. A racquet comprising:

   a) a frame; and
   b) a string according to any one of claims 1 to 33 disposed in the frame.

36. The racquet according to claim 35, wherein the racquet is one of: a tennis racquet; a badminton racquet; a squash racquet; a racquetball racquet.

37. Use of a racquet according to claim 35 or 36.

38. A method of providing a string for sporting applications comprising:

   a) providing a string or core;
   b) providing a polymeric membrane having at least some porosity
   c) filling at least some of said at least some porosity with an adhesive to form a composite; and
   d) wrapping at least a portion of the string or core with the composite.

**Patentansprüche**

1. Saite für Sportanwendungen, wobei die Saite Folgendes umfasst:

   a) eine Saite oder einen Kern; und
   b) einen Verbundstoffumfassend eine Polymermembran (1), die mindestens eine gewisse Porosität (2) aufweist, und einen Klebstoff (3), der innerhalb der mindestens einen gewissen Porosität angeordnet ist, wobei der Verbundstoff mindestens einen Teil der Saite oder des Kerns bedeckt.

2.  Saite nach Anspruch 1, wobei die mindestens eine gewisse Porosität (2) mit dem Klebstoff (3) gefüllt ist.

3.  Saite nach Anspruch 2, des Weiteren eine Klebstoffschicht (4, 5) umfassend, die auf mindestens einer Oberfläche der Polymermembran (1) angeordnet ist.

4.  Saite nach Anspruch 3, wobei die Klebstoffschicht (4, 5) diskontinuierlich ist.

5.  Saite nach Anspruch 3, wobei die Klebstoffschicht (4, 5) kontinuierlich ist.

6.  Saite nach Anspruch 1, wobei der Klebstoff (3) einen Niedertemperaturklebstoff umfasst.

7.  Saite nach Anspruch 6, wobei der Niedertemperaturklebstoff UV-ausgehärtet ist.

8.  Saite nach Anspruch 6, wobei der Niedertemperaturklebstoff des Weiteren mindestens ein Füllmaterial umfasst.

9.  Saite nach Anspruch 8, wobei das mindestens eine Füllmaterial mindestens ein Material umfasst ausgewählt aus der Gruppe bestehend aus: Keramikmaterialien, Metallen, mit Metall beschichteten Füllstoffen, metallisierten Füllstoffen, anorganischen Oxiden, Kohlenstoff, Pigmenten, Gleitmitteln und Polymeren.

10. Saite nach Anspruch 6, wobei der Niedertemperaturklebstoff mindestens ein Material umfasst, ausgewählt aus der Gruppe bestehend aus Urethanacrylaten und kationischen Epoxiden.

11. Saite nach Anspruch 1, wobei der Verbundstoff spiralförmig um mindestens einen Teil der Saite oder des Kerns gewunden ist.

12. Saite nach Anspruch 1, wobei der Verbundstoff längenmäßig um mindestens einen Teil der Saite oder des Kerns gewunden ist.

13. Saite nach Anspruch 1, wobei die Polymermembran Fluorpolymer umfasst.

14. Saite nach Anspruch 13, wobei das Fluorpolymer Schaumpolytetrafluorethylen ist.

15. Saite nach Anspruch 1, wobei der Verbundstoff eine Dicke von weniger als etwa 5 Prozent, auf den Saiten- oder Kerndurchmesser bezogen, aufweist.

16. Saite nach Anspruch 1, wobei der Verbundstoff eine Dicke von weniger als etwa 3 Prozent, auf den Saiten- oder Kerndurchmesser bezogen, aufweist.

17. Saite nach Anspruch 1, wobei der Verbundstoff eine Dicke von weniger als etwa 1 Prozent, auf den Saiten- oder Kerndurchmesser bezogen, aufweist.

18. Saite nach Anspruch 1, wobei die Saite oder der Kern Naturdarm umfasst.

19. Saite nach Anspruch 1, wobei die Saite oder der Kern ein Monofilament ist.

20. Saite nach Anspruch 1, wobei die Saite oder der Kern eine Vielzahl von Filamenten umfasst.

21. Saite nach Anspruch 20, wobei die Filamente im Wesentlichen den gleichen Durchmesser aufweisen.

22. Saite nach Anspruch 1, wobei die Saite einen Durchmesser von weniger als etwa 1,34 mm und einen dynamischen Modul von weniger als etwa 30 kN/m und eine Lebensdauer von mindestens etwa 2200 besitzt.

23. Saite nach Anspruch 1, wobei die Saite einen Durchmesser von weniger als etwa 1,25 mm und einen dynamischen Modul von weniger als etwa 30 kN/m und eine Lebensdauer von mindestens etwa 500 besitzt.

24. Saite nach Anspruch 1, wobei die Saite einen Durchmesser von weniger als etwa 1,25 mm und einen dynamischen Modul von weniger als etwa 30 kN/m und eine Lebensdauer von mindestens etwa 1000 besitzt.

**25.** Saite nach Anspruch 1, wobei die Saite einen Durchmesser von weniger als etwa 1,20 mm und einen dynamischen Modul von weniger als etwa 30 kN/m und eine Lebensdauer von mindestens etwa 500 besitzt.

**26.** Saite nach Anspruch 1, wobei die Saite einen Durchmesser von weniger als etwa 1,20 mm und einen dynamischen Modul von weniger als etwa 30 kN/m und eine Lebensdauer von mindestens etwa 800 besitzt.

**27.** Saite nach Anspruch 1, wobei Saite oder der Kern Synthesefasern umfasst.

**28.** Saite nach Anspruch 27, wobei die Synthesefasern Polyamid umfassen.

**29.** Saite nach Anspruch 28, wobei die Synthesefasern Nylon umfassen.

**30.** Saite nach Anspruch 27, wobei die Synthesefasern Polyester umfassen.

**31.** Saite nach Anspruch 27, wobei die Synthesefasern PEEK umfassen.

**32.** Saite nach Anspruch 1, wobei die Polymennembran (1) eine Membran aus Schaumpolytetrafluorethylen ist, die mindestens eine gewisse Porosität (2) aufweist, wobei der Klebstoff die mindestens eine gewisse Porosität (2) im Wesentlichen füllt, und wobei die Schlägersaite des Weiteren eine kontinuierliche Klebstoffschicht (4, 5) umfasst, die auf mindestens einer Oberfläche der Membran aus Schaumpolytetrafluorethylen (1) angeordnet ist, wobei der Verbundstoff mindestens einen Teil der Saite oder des Kerns bedeckt.

**33.** Saite nach Anspruch 1, wobei die Polymennembran (1) eine Membran aus Schaumpolytetrafluorethylen ist, die mindestens eine gewisse Porosität (2) aufweist, wobei der Klebstoff die mindestens eine gewisse Porosität (2) im Wesentlichen füllt, und wobei die Schlägersaite des Weiteren eine diskontinuierliche Klebstoffschicht (4, 5) umfasst, die auf mindestens einer Oberfläche der Membran aus Schaumpolytetrafluorethylen (1) angeordnet ist, wobei der Verbundstoff mindestens einen Teil der Saite oder des Kerns bedeckt.

**34.** Saite nach einem der Ansprüche 1 bis 33, wobei die Saite zur Verwendung bei einem Schläger konfiguriert ist.

**35.** Schläger umfassend:

a) einen Rahmen; und
b) eine Saite nach einem der Ansprüche 1 bis 33, die in dem Rahmen angeordnet ist.

**36.** Schläger nach Anspruch 35, wobei der Schläger einer der Folgenden ist: ein Tennisschläger; ein Badmintonschläger; ein Squashschläger; ein Raquetballschläger.

**37.** Verwendung eines Schlägers nach Anspruch 35 oder 36.

**38.** Methode für das Bereitstellen einer Saite für Sportanwendungen, umfassend:

a) das Bereitstellen einer Saite oder eines Kerns;
b) das Bereitstellen einer Polymermembran, die mindestens eine gewisse Porosität aufweist;
c) das mindestens teilweise Füllen der einen gewissen Porosität mit einem Klebstoff unter Bildung eines Verbundstoffs; und
d) das Umwickeln mindestens eines Teils der Saite oder des Kerns mit dem Verbundstoff.

**Revendications**

**1.** Corde pour des applications sportives, la corde comprenant:

a) une corde ou une âme; et
b) un composite comprenant une membrane polymère (1) ayant au moins une certaine porosité (2) et un adhésif (3) disposés à l'intérieur de la dite au moins certaine porosité, le composite recouvrant au moins une portion de ladite corde ou de ladite âme.

**2.** Corde selon la revendication 1, dans laquelle ladite au moins une certaine porosité (2) est remplie de l'adhésif (3).

**3.** Corde selon la revendication 2, comprenant en outre une couche adhésive (4, 5) disposée sur la au moins une surface de ladite membrane polymère (1).

**4.** Corde selon la revendication 3, dans laquelle la couche adhésive (4, 5) est discontinue.

**5.** Corde selon la revendication 3, dans laquelle la couche adhésive (4, 5) est continue.

**6.** Corde selon la revendication 1, dans laquelle l'adhésif (3) comprend un adhésif basse température.

**7.** Corde selon la revendication 6, dans laquelle l'adhésifbasse température est durci par rayonnement UV.

**8.** Corde selon la revendication 6, dans laquelle l'adhésif basse température comprend en outre au moins un matériau de type charge.

**9.** Corde selon la revendication 8, dans laquelle le au moins un matériau de type charge comprend au moins un matériau choisi parmi le groupe constitué des : céramiques, métaux, charges revêtues de métaux, charges métallisées, oxydes inorganiques, du carbone, des pigments, lubrifiants et polymères.

**10.** Corde selon la revendication 6, dans laquelle l'adhésif basse température comprend au moins un matériau choisi parmi le groupe constitué des acrylates d'uréthane et des époxys cationiques.

**11.** Corde selon la revendication 1, dans laquelle le composite est enveloppé de manière hélicoïdale autour d'au moins une portion de la corde ou de l'âme.

**12.** Corde selon la revendication 1, dans laquelle le composite est enveloppé de manière longitudinale autour d'au moins une portion de la corde ou de l'âme.

**13.** Corde selon la revendication 1, dans laquelle la membrane polymère comprend un fluoropolymère.

**14.** Corde selon la revendication 13, dans laquelle le fluoropolymère est du polytétrafluoroéthylène expansé.

**15.** Corde selon la revendication 1, dans laquelle le composite a une épaisseur inférieure à environ 5 pour cent du diamètre de la corde ou de l'âme.

**16.** Corde selon la revendication 1, dans laquelle le composite a une épaisseur inférieure à environ 3 pour cent du diamètre de la corde ou de l'âme.

**17.** Corde selon la revendication 1, dans laquelle le composite a une épaisseur inférieure à environ 1 pour cent du diamètre de la corde ou de l'âme.

**18.** Corde selon la revendication 1, dans laquelle la corde ou l'âme comprend du boyau naturel.

**19.** Corde selon la revendication 1, dans laquelle la corde ou l'âme est un monofilament.

**20.** Corde selon la revendication 1, dans laquelle la corde ou l'âme comprend une pluralité de filaments.

**21.** Corde selon la revendication 20, dans laquelle lesdits filaments sont substantiellement du même diamètre.

**22.** Corde selon la revendication 1, la corde ayant un diamètre inférieur à environ 1,34 mm et ayant un module dynamique inférieur à environ 30 kN/m et une durabilité d'au moins environ 2200.

**23.** Corde selon la revendication 1, la corde ayant un diamètre inférieur à environ 1,25 mm et ayant un module dynamique inférieur à environ 30 kN/m et une durabilité d'au moins environ 500.

**24.** Corde selon la revendication 1, la corde ayant un diamètre inférieur à environ 1,25 mm et ayant un module dynamique inférieur à environ 30 kN/m et une durabilité d'au moins environ 1000.

**25.** Corde selon la revendication 1, la corde ayant un diamètre inférieur à environ 1,20 mm et ayant un module dynamique inférieur à environ 30 kN/m et une durabilité d'au moins environ 500.

**26.** Corde selon la revendication 1, la corde ayant un diamètre inférieur à environ 1,20 mm et ayant un module dynamique inférieur à environ 30 kN/m et une durabilité d'au moins environ 800.

**27.** Corde selon la revendication 1, dans laquelle la corde ou l'âme comprend des fibres synthétiques.

**28.** Corde selon la revendication 27, dans laquelle les fibres synthétiques comprennent du polyamide.

**29.** Corde selon la revendication 28, dans laquelle les fibres synthétiques comprennent du nylon.

**30.** Corde selon la revendication 27, dans laquelle les fibres synthétiques comprennent du polyester.

**31.** Corde selon la revendication 27, dans laquelle les fibres synthétiques comprennent du PEEK.

**32.** Corde selon la revendication 1, dans laquelle la membrane polymère (1) est une membrane en polytétrafluoroéthylène expansé ayant au moins une certaine porosité (2), l'adhésif remplissant substantiellement ladite au moins certaine porosité (2), et dans laquelle la corde de raquette comprend en outre une couche adhésive continue (4, 5) disposée sur au moins une surface de ladite membrane de polytétrafluoroéthylène expansé (1), ledit composite recouvrant au moins une portion de ladite corde ou âme.

**33.** Corde selon la revendication 1, dans laquelle la membrane polymère (1) est une membrane en polytétrafluoroéthylène expansé ayant au moins une certaine porosité (2), l'adhésif remplissant substantiellement ladite au moins certaine porosité (2), et dans laquelle la corde de raquette comprend en outre une couche adhésive discontinue (4,5) disposée sur au moins une surface de ladite membrane de polytétrafluoroéthylène expansé, ledit composite recouvrant au moins une portion de ladite corde ou âme.

**34.** Corde selon l'une quelconque des revendications 1 à 33, dans laquelle la corde est configurée pour l'utilisation avec une raquette.

**35.** Raquette comprenant:

a) un cadre; et
b) une corde selon l'une quelconque des revendications 1 à 33 disposée dans le cadre.

**36.** Raquette selon la revendication 35, dans laquelle la raquette est une raquette parmi: une raquette de tennis; une raquette de badminton; une raquette de squash; une raquette de racquetball.

**37.** Utilisation d'une raquette selon la revendication 35 ou 36.

**38.** Procédé de fourniture d'une corde pour des applications sportives comprenant:

a) la fourniture d'une corde ou âme;
b) la fourniture d'une membrane polymère ayant au moins une certaine porosité;
c) le remplissage au moins partiellement de ladite au moins certaine porosité avec un adhésif pour former un composite; et
d) l'enveloppement d'au moins une portion de la corde ou de l'âme avec le composite.

15

FIG. 1

22

20

Fig. 2

1

2

3

Fig. 3

2

3

Fig. 4

1

2

3

4

Fig. 5

3

2

4

Fig. 6

Fig. 7

Fig 8a

10

12

12

8 t

4

9a

11

12

11

12

9 b

Fig. 10

# Fig. 11

Fig. 12

46

48

42'

42

40

44

44'

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2001035002 A, Carr
- US 3953566 A
- US 3962153 A
- US 4096227 A
- US 4187390 A